(19) Europäisches Patentamt
European Patent Office
Office européen des brevets

(11) Numéro de publication: **0 005 094**
**B1**

(12) **FASCICULE DE BREVET EUROPEEN**

(21) Numéro de dépôt: **79400213.9**

(22) Date de dépôt: **03.04.79**

(51) Int. Cl.³: **C 07 C 93/04**

(54) **Procédé de préparation de tris-(polyoxaalkyl)-amines**

(30) Priorité: **21.04.78 FR 7811824**

(43) Date de publication de la demande:
**31.10.79 Bulletin 79/22**

(45) Mention de la délivrance du brevet:
**29.10.80 Bulletin 80/22**

(84) Etats contractants désignés:
**BE CH DE FR GB IT NL SE**

(56) Documents cités:
**US - A - 2 293 494**
**CHEMICAL ABSTRACTS, Vol. 74, n° 19, 10 mai 1971,**
**Réf. 100001x, page 509**
**COLUMBUS (OHIO) USA**
**CHEMICAL ABSTRACTS, Vol. 80, n° 22, 3 juin 1974**
**Réf. 122681v, pages 107—108**
**COLUMBUS (OHIO) USA**
**JOURNAL OF THE AMERICAN CHEMICAL SOCIETY,**
**Vol. 70(9), septembre 1948 WASHINGTON D.C. (US)**
**J. B. WRIGHT: "Histamine antagonists alkamine ethers",**
**pages 3098—3100**

(73) Titulaire: **RHONE-POULENC INDUSTRIES**
**22, avenue Montaigne**
**F - 75008 Paris (FR)**

(72) Inventeur: **Soula Gérard**
**33, rue Nungesser**
**F - 69330 Meyzieu (FR)**

(74) Mandataire: **Fabre, Madeleine-France et al**
**RHONE POULENC Service Brevets**
**Chimie et Polymères B.P. 753**
**F - 75360 Paris Cedex 08 (FR)**

**EP 0 005 094 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé de préparation de tris (polyoxaalkyl) amines

La présente invention a pour objet un nouveau procédé préparation de tris(polyoxaalkyl)amines.

Il est connu notamment d'après le brevet français 1.302.365 de préparer des alcoxyéthylèneamines de formule:

$$\text{(A)} \quad R\left[-(O - CH_2 - CH_2)_n\right] N\ Z_{3-m}$$

—R représente un groupe alcoyle en $C_1$—$C_8$
—n représente un nombre entier de 1 à 3
—m représente un nombre entier de 1 à 3
—Z représente de l'hydrogène ou un radical alcoyle inférieur, par réaction d'un éther d'éthylèneglycol de formule:

$$R-(O - CH_2 - CH_2-)_nOH,$$

sur une amine en présence d'hydrogène et d'un catalyseur d'hydrogénation/déshydrogénation, ladite réaction étant réalisée en phase vapeur à une température de 150 à 250°C.

Un tel procédé présente l'inconvénient majeur de ne pas être sélectif, de ne permettre l'obtention que de mélanges de produits répondant à la formule (A); de plus, on constate que les taux de conversion en tris(polyoxaalkyl)amines de formule:

$$\left[R(-O - CH_2 - CH_2)_n\right]_3 N$$

sont très faibles.

La demanderesse a trouvé un nouveau procédé permettant d'obtenir avec une grande sélectivité des tris(polyoxaalkyl)amines de formule:

$$N\left[CH_2 - CH_2 - O -(CH_2 - CH_2 - O)_x - R\right]_3$$

où R représente un radical alcoyle en $C_1$—$C_8$, de préférence en $C_1$—$C_4$ et x représente un nombre entier compris l et 4, de préférence égal à 1 ou 2.

Le procédé objet de l'invention est caractérisé en ce que l'on condense un sel de métal alcalin d'un monoalcoyléther d'un éthylèneglycol de formule:

$$\text{(I)} \quad R-(O - CH_2 - CH_2-)_xO - M$$

où R représente un radical alcoyle en $C_1$—$C_8$, de préférence en $C_1$—$C_4$, x représente un nombre entier compris entre 1 et 4, de préférence égal à 1 ou 2 et M représente un atome de métal alcalin choisi parmi le sodium, le potassium et le lithium, sur une tris(halogénoalkyl)amine choisie parmi le chlorhydrate de tris(chloro-2 éthyl)amine, la tris(chloro-2 éthyl)amine ou la tris(bromo-2 éthyl)amine, selon un rapport molaire sel de métal alcalin de formule (I) /"tris(halogénoalkyl)amine" compris entre 3 et 5, puis en ce que l'on sépare la tris(polyoxaalkyl)amine obtenue de formule:

$$N\left[CH_2 - CH_2 - O -(CH_2 - CH_2 - O)_x - R\right]_3$$

Pour une bonne réalisation de l'invention, l'opération de condensation est réalisée à une température comprise entre 80 et 200°C, de préférence entre 100 et 150°C pendant 0,5 à 20 heures, de préférence pendant 1 à 15 heures, en présence d'un solvant. Parmi les solvants pouvant être utilisés, on peut citer le chlorobenzène, l'orthodichlorobenzène ... et de préférence le monoalcoyléther d'éthylèneglycol de formule (II):

$$R-(O - CH_2 - CH_2-)_xO - H$$

correspondant au sel de métal alcalin de monoalcoyléther d'éthylèneglycol de formule (I), en quantité telle que l'on ait une solution contenant de 0,5 à 5 moles de sel de métal alcalin de formule (I), de préférence de 2 à 5 moles par litre de monoalcoyléther d'éthylèneglycol de formule (II).

Lorsque la "tris(halogénoéthyl)amine" mise en oeuvre est la tris(bromo-2 éthyl)amine ou la tris(chloro-2 éthyl)amine, le rapport molaire sel de métal alcalin de formule (I) / tris(bromo-2 éthyl)amine ou tris(chloro-2 éthyl)amine est de préférence compris entre 3 et 4.

Lorsque la tris(halogénoéthyl)amine mise en oeuvre est le chlorhydrate de tris(chloro-2 éthyl)amine, le rapport molaire sel de métal alcalin de formule (I) /chlorhydrate est de préférence compris entre 4 et 5.

Le schéma de la réaction de condensation par exemple avec le chlorhyrate de tris(chloro-2 éthyl)amine est le suivant:

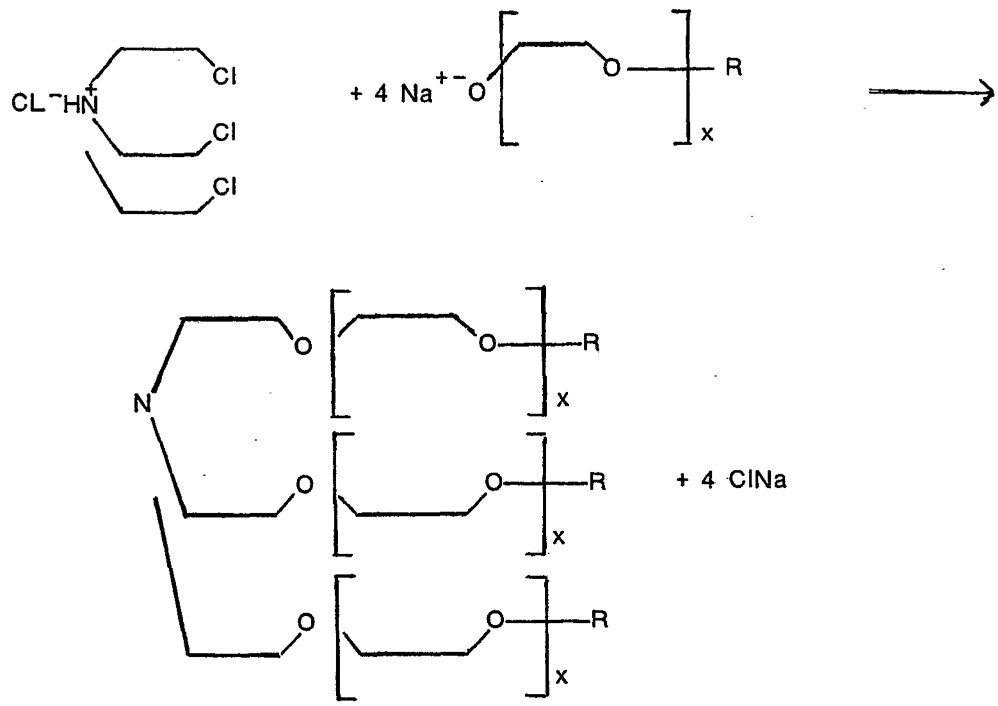

Les solutions de sels de métaux alcalins de formule (I) dans le monoalcoyléther d'éthylèneglycol de formule (II), à mettre en oeuvre, peuvent être préparées d'une manière simple et sélective à partir de sodium ou de potassium (ou de leur hydroxyde correspondant) et de monoacloyléther d'éthylèneglycol de formule (II) selon des quantités correspondant à 0,5 à 5 atomes grammes de sodium ou de potassium, de préférence de 2 à 5 atomes-grammes pour un litre de monoalcoyléther d'éthylèneglycol de formule (II). La préparation desdites solutions peut être réalisée à tempèraturer ambiante à partir de sodium ou de potassium, ou à une température supérieure à celle de distillation de l'eau, à partir de soude ou de potasse.

Le chlorhydrate de tris(chloro-2 éthyl)amine à mettre en oeuvre peut être préparé d'une manière connue à partir de chlorhydrate de triéthanolamine et de chlorure de thionyle, selon la méthode décrite par K. WARD dans JACS 57, 914, 1935 ou par J. P. MASON et D. J. GASCH dans JACS 60, 2816, 1938.

La tris(chloro-2 éthyl)amine à mettre en oeuvre peut être préparée par décomposition du chlorhydrate de tris(chloro-2 éthyl)amine par du bicarbonate de sodium.

Le tris(bromo-2 éthyl)amine à mettre en oeuvre peut être préparé d'une manière connue à partir de la triéthanolamine et du tribromure de phosphore.

Les exemples suivants sont donnés à titre indicatif et ne peuvent être considérés comme une limite du domaine et de l'esprit de l'invention.

## Exemple 1

Préparation de la tris(dioxa-3,6 heptyl)amine

*Préparation du méthoxy-2 éthanolate de sodium*

Dans un ballon tricol d'un litre, muni d'un agitateur mécanique, d'un thermomètre, d'un réfrigérant, on introduit 380 g de méthoxy-2 éthanol (5 moles). On ajoute 23 g de sodium (1 mole) en 3 heures en maintenant la température du mélange à 40°C.

*Synthèse de la tris(dioxa-3,6 heptyl)amine*

Au mélange précédent, on ajoute 51,6 g de chlorhydrate de tris(chloro-2 éthyl)amine, soit 0,215 mole. On chauffe alors le mélange à reflux du méthoxy-2 éthanol ($\tau$ = 125°C) pendant 12 heures, puis on distille le solvant sous pression réduite. Le méthoxy-2 éthanolate de sodium en excès est neutralisé par addition de 11,6 $cm^3$ d'HCl aqueux (10N). Le chlorure de sodium est filtré et la solution est distillée.

La tris(dioxa-3,6 heptyl)amine distille entre 165°C et 180°C sous 66,5 Pa (soit 0,5 mm Hg). On obtient 49 g de produit soit un rendement de 70% L'analyse élémentaire est la suivante:

| | Mesuré % | Théorie % |
|---|---|---|
| C | 55,7 | 55,7 |
| O | 30,2 | 29,7 |
| N | 4,3 | 4,3 |
| H | 10,1 | 10,2 |

Exemple 2

*Synthèse de la tris(dioxa-3,6 décyl)amine*

Dans un ballon tricol de 1 litre décrit à l'exemple 1, on introduit 590 g de oxa-3 heptanol-1 (monoéther butylique de l'éthylèneglycol). On ajoute 40 g de soude en pastille puis on chauffe le mélange à 120°C. Il se forme alors l'oxa-3 heptanolate de sodium et de l'eau qui distille.

Lorsque toute l'eau de la réaction est éliminée, on introduit 55 g de chlorhydrate de tris(chloro-2 éthyl)amine. Le mélange est chauffé à 130°C pendant 5 heures puis refroidi et l'excès d'alcoolate de sodium neutralisé par une solution aqueuse d'acide chlorhydrique à 10%. Après élimination de l'oxa-3 heptanol par distillation et du chlorure de sodium par filtration, la tris(dioxa-3,6 décyl)amine est distillée ($\tau$ = 192°C sous 13,3 Pa, soit 0,1 mmHg).

L'analyse élémentaire du produit obtenu est la suivante:

| | Mesuré % | Théorie % |
|---|---|---|
| C | 64,06 | 64,14 |
| O | 21,39 | 21,38 |
| N | 3,15 | 3,12 |
| H | 11,40 | 11,35 |

Exemple 3

*Synthèse de la tris(trioxa-3,6,9 décyl)amine*

Dans un ballon tricol de 1 litre équipé d'un agitateur mécanique, d'un condenseur et d'un thermomètre, on introduit 600 g d'éther monométhylique de diéthylèneglycol (dioxa-3,6 heptanol-1) soit 5 moles puis 23 g de sodium (1 mole) par petites fractions afin de former le dioxa-3,6 heptanolate de sodium.

Lorsque le sodium est totalement transformé, on ajoute alors 51,8 g de chlorhydrate de la tris(chloro-2 éthyl)amine (soit 0,215 mole). Le mélange est chauffé à 130°C pendant 8 heures sous agitation puis refroidi et l'excès d'alcoolate de sodium neutralisé par une solution aqueuse d'acide chlorhydrique à 10%. Le dioxa-3,6 heptanol-1 est éliminé par distillation à 130°C sous 2666 Pa (suit 20 mmHg). Le mélange obtenu est filtré afin d'éliminer le chlorure de sodium puis le produit est distillé. On obtient ainsi 83 g de tris(trioxa-3,6,9 décyl)amine qui distille à 189°C sous 13,3 Pa (soit 0,1 mmHg).

L'analyse élémentaire du produit obtenu est la suivante:

| | Mesuré % | Théorie % |
|---|---|---|
| C | 55,36 | 55,38 |
| H | 9,82 | 9,89 |
| N | 3,09 | 3,08 |
| O | 31,73 | 31,65 |

**Revendications**

1. Procédé de préparation de tris(polyoxaalkyl)amines de formule:

$$N\left[CH_2-CH_2-O\left(CH_2-CH_2-O\right)_x R\right]_3$$

où R représente un radical alcoyle en $C_1$—$C_8$ et x représente un nombre entier compris entre 1 et 4, caractérisé en ce que l'on condense un sel de métal alcalin d'un monoalcoyléther d'un éthylèneglycol de formule:

$$R\left(O-CH_2-CH_2\right)_x O-M$$

où M représente un atome de métal alcalin choisi parmi le sodium, le potassium et le lithium, sur une "tris(halogénoéthyl)amine" choisie parmi le chlorhydrate de tris(chloro-2 éthyl)amine, la tris(chloro-2 éthyl)amine ou la tris(bromo-2 éthyl)amine, selon un rapport molaire sel de métal alcalin de formule (I)/"tris(halogénoéthyl)amine" compris entre 3 et 5.

2. Procédé selon la revendication 1 charactérisé en ce que R représente un radical alcoyle en $C_1$—$C_4$ et x un nombre entier égal à 1 ou 2.

3. Procédé selon la revendication 1 ou la revendication 2 caractérisé en ce que l'étape de condensation est réalisée à une température comprise entre 80 et 200°C pendant 0,5 à 20 heures.

4. Procédé selon la revendication 3 caractérisé en ce que l'étape de condensation est réalisée à une température comprise entre 100 et 150°C pendant 1 à 15 heures.

5. Procédé selon l'une quelconque des revendications 1 à 4 caractérisé en ce que l'étape de condensation est réalisée en présence de monoalcoyléther d'éthylèneglycol de formule (II):

$$R\left(O-CH_2-CH_2\right)_x OH$$

correspondant au sel de métal alcalin de formule (I) et en ce que la quantité de sel de formule I présente est de 0,5 à 5 moles par litre de monoalcoyléther de formule II.

6. Procédé selon la revendication 5 caractérisé en ce que la quantité de sel de formule I présente est de 2 à 5 moles par litre de monoalcoyléther de formule II.

7. Procédé selon l'une quelconque des revendications précédentes caractérisé en ce que la tris(halogénoéthyl)amine mise en oeuvre est la tris(chloro-2 éthyl)amine ou la tris(bromo-2 éthyl)amine, et en ce que le rapport molaire sel de formule I/tris(halogénoéthyl)amine est compris entre 3 et 4.

8. Procédé selon l'une quelconque des revendications 1 à 6 caractérisé en ce que la tris(halogénoéthyl)amine mise en oeuvre est le chlorhydrate de tris(chlor-2 éthyl)amine et en ce que le rapport molaire sel de formule I/chlorhydrate de tris(chloro-2 éthyl)amine est compris entre et 5.

**Claims**

1. Process for the preparation of tris-(polyoxaalkyl)-amines of the formula:

$$N\left[CH_2-CH_2-O\left(CH_2-CH_2-O\right)_x R\right]_3$$

in which R represents a $C_1$—$C_8$ alkyl radical and x represents an integer between 1 and 4, characterised in that an alkali metal salt of an ethylene glycol monoalkyl ether, of the formula (I):

$$R\left(O-CH_2-CH_2\right)_x O-M$$

in which M represents an alkali metal atom chosen from amongst sodium, potassium and lithium, is condensed with a "tris-(halogenoethyl)-amine" chosen from amongst tris-(2-chloroethyl)-amine hydrochloride, tris-(2-chloroethyl)-amine or tris-(2-bromoethyl)-amine, in a molar ratio alkali metal salt of the formula (I)/"tris-(halogenoethyl)-amine" of between 3 and 5.

2. Process according to Claim 1, characterised in that R represents a $C_1$—$C_4$ alkyl radical and x represents an integer equal to 1 or 2.

3. Process according to Claim 1 or Claim 2, characterised in that the condensation step is carried out at a temperature between 80 and 200°C for 0.5 to 20 hours.

4. Process according to Claim 3, characterised in that the condensation step is carried out at a temperature between 100 and 150°C for 1 to 15 hours.

5. Process according to any one of Claims 1 to 4, characterised in that the condensation step is carried out in the presence of an ethylene glycol monoalkyl ether of the formula (II):

$$R\text{-}(O-CH_2-CH_2)_x OH$$

corresponding to the alkali metal salt of the formula (I), and in that the amount of salt of the formula I present is from 0.5 to 5 mols per litre of monoalkyl ether of the formula II.

6. Process according to Claim 5, characterised in that the amount of salt of the formula I present is from 2 to 5 moles per litre of monoalkyl ether of the formula II.

7. Process according to any one of the preceding claims, characterised in that the tris-(halogenoethyl)-amine employed is tris(2-chloroethyl)-amine or tris-(2-bromoethyl)-amine, and in that the molar ratio salt of the formula I/tris-(halogenoethyl)-amine is between 3 and 4.

8. Process according to any one of Claims 1 to 6, characterised in that the tris-(halogenoethyl)-amine employed is tris-(2-chloroethyl)-amine hydrochloride, and in that the molar ratio salt of the formula I/tris-(2-chloroethyl)-amine hydrochloride is between 4 and 5.

**Patentansprüche**

1. Verfahren zur Herstellung von Tris(polyoxaalkyl)-aminen der Formel

$$N\left[CH_2-CH_2-O-(CH_2-CH_2-O)_x R\right]_3$$

in der R eine $C_1$—$C_8$ Alkylgruppe und x eine ganze Zahl von 1 bis 4 bedeutet, dadurch gekennzeichnet, daß man ein Alkalisalz eines Monoalkyläthers eines Äthylenglykols der Formel I

$$R\text{-}(O-CH_2-CH_2)_x O-M$$

in der M ein Alkaliatom ausgewählt unter Natrium, Kalium und Lithium ist, mit einem "Tris(halogenäthyl)amin" ausgewählt unter Tris(2-chloräthyl)amin-chlorhydrat, Tris(2-chloräthyl)amin oder Tris(2-bromäthyl)amin kondensiert, in einem Molverhältnis von Alkalisalz der Formel (I) zu "Tris(halogenäthyl)amin" zwischen 3 bis 5.

2. Verfahren nach Anspruch 1, dadurch gekennzeichnet, daß R eine $C_1$—$C_4$ Alkylgruppe bedeutet und x die ganze Zahl 1 oder 2 ist.

3. Verfahren nach Anspruch 1 oder 2, dadurch gekennzeichnet, daß die Kondensationsstufe bei einer Temperatur zwischen 80 und 200°C während, 0,5 bis 20 Stunden durchgeführt wird.

4. Verfahren nach Anspruch 3, dadurch gekennzeichnet, daß die Kondensationsstufe bei einer Temperatur zwischen 100 und 150°C während 1 bis 15 Stunden durchgeführt wird.

5. Verfahren nach einem der Ansprüche 1 bis 4, dadurch gekennzeichnet, daß die Kondensationsstufe durchgeführt wird in Gegenwart von dem Alkalisalz der Formel (I) entsprechenden Äthylen-glykol-monoalkyläther der Formel (II)

$$R\text{-}(O-CH_2-CH_2)_x OH-M$$

und daß die Menge Salz der Formel I 0,5 bis 5 Mol je Liter Monoalkyläther der Formel II ausmacht.

6. Verfahren nach Anspruch 5, dadurch gekennzeichnet, daß die Menge des Salzes der Formel I 2 bis 5 Mol je Liter Monoalkyläther der Formel II ausmacht.

7. Verfahren nach einem der vorangehenden Ansprüche, dadurch gekennzeichnet, daß das eingesetzte Tris(halogenäthyl)amin, das Tris(2-chloräthyl)amin oder das Tris(2-bromäthyl)amin ist und daß das Molverhältnis von Salz der Formel I zu Tris(halogenäthyl)amin zwischen 3 und 4 liegt.

8. Verfahren nach einem der Ansprüche 1 bis 6, dadurch gekennzeichnet, daß das eingesetzte Tris(halogenäthyl)amin, das Tris(2-chloräthyl)amin-chlorhydrat ist und daß das Molverhältnis von Salz der Formel I zu Tris(2-chloräthyl)amin zwischen 4 und 5 liegt.